Europäisches Patentamt

European Patent Office

Office européen des brevets

(19)

(11) Numéro de publication : **0 457 640 B1**

(12) **FASCICULE DE BREVET EUROPEEN**

(45) Date de publication du fascicule du brevet :
12.01.94 Bulletin 94/02

(51) Int. Cl.⁵ : **C07C 15/00, C07C 2/00**

(21) Numéro de dépôt : **91401155.6**

(22) Date de dépôt : **29.04.91**

(54) **Utilisation d'un catalyseur contenant une zéolithe, un métal noble de la famille du platine et un métal additionnel dans l'aromatisation des hydrocarbures contenant de 2 à 4 atomes de carbone par molécule.**

(30) Priorité : **11.05.90 FR 9006015**

(43) Date de publication de la demande :
**21.11.91 Bulletin 91/47**

(45) Mention de la délivrance du brevet :
**12.01.94 Bulletin 94/02**

(84) Etats contractants désignés :
**DE ES GB IT NL**

(56) Documents cités :
**EP-A- 0 351 311
EP-A- 0 351 312
GB-A- 1 496 379
US-A- 3 296 324**

(73) Titulaire : **INSTITUT FRANCAIS DU PETROLE
4, Avenue de Bois Préau
F-92502 Rueil-Malmaison (FR)**

(72) Inventeur : **Raatz, Francis
25, rue de la Carrière
F-57500 Saint Avold (FR)**
Inventeur : **Juguin, Bernard
46, avenue du Stade
F-92500 Rueil Malmaison (FR)**
Inventeur : **Bournonville, Jean-Paul
43, rue des Groues Vauréal
F-95000 Cergy Pontoise (FR)**

## Description

La présente invention concerne l'utilisation d'un catalyseur composite comprenant d'une part une zéolithe de structure MFI contenant du silicium et de l'aluminium et d'autre part un métal de la famille du platine déposé sur un support oxyde réfractaire sur lequel est ajouté au moins un additif métallique choisi dans le groupe constitué par l'étain, le germanium, l'indium et le plomb, dans les réactions d'aromatisation des hydrocarbures comprenant entre 2 et 4 atomes de carbones par molécule.

L'aromatisation de produits légers par un catalyseur comprenant un métal sur un support est connue : l'aromatisation de paraffines $C_3$-$C_5$ par un catalyseur comprenant un métal du groupe VIII sur un support est décrite dans le brevet US-A-3,296,324 ; l'aromatisation d'une charge $C_4$ par un catalyseur comprenant du germanium et/ou de l'indium et/ou de l'étain sur un support est décrite dans la demande de brevet GB-A-1.496.379.

La réaction d'aromatisation du propane et du butane en présence d'un catalyseur contenant de la zéolithe ZSM5 (ou MFI) et du gallium a été découvert et breveté par la société British Petroleum (US-A 4180689). D'autres sociétés ou organismes ont depuis cette date déposé des brevets relatifs à des modifications du solide (US-A-4795844 ou Brevet Européen EP-B-0252705) et/ou à des changements de charge : coupe $C_2$-$C_{12}$ (Brevet Européen EP-B-0252705), éthane et éthylène (Brevet Européen EP-B-0050021 et US-A-4350835). Le mode d'introduction du gallium a fait également l'objet de prise de brevets (Brevets Européens EP-B-0120018 et 0184927). La demande de brevet EP-A-351.311 décrit un catalyseur comprenant une matrice et un gallosilicate de structure MFI, et son utilisation en aromatisation des gaz légers $C_2$-$C_4$.

L'ajout d'un autre métal au système Ga/MFI a été aussi envisagé pour améliorer les sélectivités en aromatiques et réduire la quantité de coke sur le catalyseur.

Ainsi l'imprégnation d'un catalyseur Ga/MFI par du rhénium, associé au platine ou au palladium permet d'améliorer de façon signicative la sélectivité de production d'aromatiques (US-A-4766265). D'autres brevets revendiquent l'addition de platine et de palladium au catalyseur Ga-MFI (US-A-4407728, Brevets Européens EP-B-0215579, 0216491, 0224162, 0228267 et Brevets Japonais 61268634, 62081329, 612277636).

L'addition de platine à la zéolithe MFI permet d'améliorer la conversion du propane (T. INUI, F. OKAZUMI, Y. MAKINO Chem Express 1 (1), 53-56- 1985). Cependant la sélectivité de production de méthane et d'éthane est sensiblement augmentée. L'adjonction de rhénium au platine accentue encore la production de méthane et d'éthane par hydrogénolyse (S. ENGELS et coll, Catalysis Today 3, 437-443, 1988). L'ajout de cuivre (P. MERIAUDEAU et coll, Zeolites : Facts, figures, Future, 1423-29, 1989) et de chrome (E.S SHPIRO et coll. International Symposium on Zeolites as Catalysts, Sorbents and Detergent builders, Würzburg (RFA), p. 73, 1988) diminue la production de méthane, mais la sélectivité en aromatiques reste inférieure aux systèmes Ga/MFI. Par ailleurs, très récemment l'addition de soufre à un catalyseur Pt/MFI permet d'en améliorer sensiblement la sélectivité de production d'aromatiques à partir de paraffines contenant de 6 à 12 atomes de carbones (USP-A-4835336).

On a découvert que des performances améliorées par rapport à ce qui est connu dans l'art antérieur peuvent être obtenues en aromatisation des hydrocarbures légers en utilisant un catalyseur composite particulier.

Ce catalyseur comprend une zéolithe de structure MFI d'une part, et d'autre part un support généralement amorphe sur lequel est déposé au moins un métal noble de la famille du platine et au moins un métal additionnel choisi dans le groupe constitué par l'étain, le germanium, le plomb, et l'indium.

La zéolithe de structure MFI constituant une partie du catalyseur de la présente invention, peut être préparée par toutes les techniques connues dans l'art antérieur. La synthèse de la-dite zéolithe MFI peut être réalisée en milieu classique OH⁻, en présence ou non de structurants organiques et/ou d'alcool. La synthèse de la zéolithe MFI en milieu OH⁻ selon les techniques connues dans l'art antérieur est largement décrite dans le document suivant : Synthesis of High Silica Zeolites, P. Jacobs and J. Martens, Studies in Surface Science and Catalysis, Volume 33, Elsevier editor, 1987. La zéolithe MFI peut également être synthétisée dans des milieux moins classiques comme par exemple le milieu fluorure (Brevet Européen EP-A-172068 de CFR).

Après synthèse, la zéolithe MFI est transformée en une forme hydrogène par élimination totale ou partielle des composés organiques et/ou des cations alcalins ou alcalino-terreux qu'elle contient éventuellement après la synthèse. Toutes les techniques connues dans l'art antérieur peuvent être utilisées pour passer à la forme hydrogène, comme par exemple les calcinations sous atmosphère oxydante ou non, les échanges ioniques suivis ou non de calcination, les traitements chimiques divers etc.

Toutes les zéolithes MFI synthétisées dans le système Si-Al conviennent pour la présente invention. Cependant leur rapport Si/Al sera supérieur à 7, de préférence supérieur à 25 et de préférence compris entre 40 et 200.

Les supports des métaux ajoutés à la zéolithe sont généralement choisis parmi les oxydes des métaux des groupes II, III et/ou IV de la classification périodique des éléments, tels que par exemple, les oxydes de magnésium, d'aluminium, de titane, de zirconium, de thorium ou de silicium, pris seuls ou en mélange entre eux ou avec des oxydes d'autres éléments de la classification périodique, tels que par exemple le bore. On peut aussi utiliser du charbon.

Le support préféré est l'alumine ; la surface spécifique de l'alumine peut avantageusement être comprise entre 50 et 600 m² par gramme, de préférence entre 150 et 400 m²/g.

Le catalyseur composite de la présente invention peut être préparé suivant deux voies dont le principe est donné ci-après.

Première voie : mélange de la zéolithe MFI avec le support ; ce mélange peut être réalisé entre deux poudres, entre les deux solides mis en forme, entre une poudre et un des solides mis en forme. On peut également mettre en forme conjointement les deux solides par toutes les techniques connues dans l'art antérieur : pastillage, extrusion, dragéification, coagulation en goutte, séchage par atomisation. Lors de ces opérations de mise en forme, on pourra si nécessaire ajouter un additif de mise en forme (silice etc). Après mélange et/ou mise en forme, on procède au dépôt des divers agents actifs sur le support (en présence donc de la zéolithe).

Deuxième voie : on dépose préalablement les agents actifs sur le support, que l'on mélange ou que l'on met en forme avec la zéolithe MFI dans les mêmes conditions que précédemment. Dans une variante, la zéolithe pourra être introduite dans le catalyseur composite à l'une quelconque des étapes de dépôt des agents actifs sur le support.

La méthode préférée de préparation consiste à déposer les agents actifs sur le support puis à introduire la zéolithe dans le catalyseur final par mise en forme des deux poudres. La mise en forme sera de préférence réalisée après un broyage micronique, qui pourra être réalisée en utilisant la technique de broyage humide.

Le catalyseur composite contient entre 1 et 99 % en poids de zéolithe, le complément à 100% étant constitué par le support chargé en différents agents actifs. La proportion respective de zéolithe et de support varie dans une large gamme, car elle dépend d'une part du rapport Si/Al de la zéolithe et d'autre part de la teneur en agent actifs du support.

La partie du catalyseur composite comprenant du métal noble est généralement préparée selon des méthodes classiques consistant à imprégner le support au moyen de solutions de composés des métaux que l'on désire introduire. On utilise soit une solution commune de ces métaux, soit des solutions distinctes pour le métal de la famille du platine et pour le métal M additionnel. Quand on utilise plusieurs solutions, on peut procéder à des séchages et/ou calcinations intermédiaires. On termine habituellement par une calcination par exemple entre environ 500 et 1000°C, de préférence en présence d'oxygène libre, par exemple en effectuant un balayage d'air.

La partie du catalyseur composite, contenant le métal noble renferme en poids par rapport au support (a) environ 0,01 à 2 % et plus particulièrement environ 0,1 à 0,5 % d'au moins un métal noble de la famille du platine, le platine étant toujours présent, (b) environ 0,005 à 0,60 %, et de préférence 0,01 à 0,50 d'étain ou 0,005 à 0,40 % de préférence environ 0,01 à 0,6 % et plus particulièrement 0,02 à 0,50 % d'au moins un métal choisi dans le groupe constitué par le germanium, le plomb, et l'indium.

Lorsqu'il y a au moins deux métaux de la famille étain, germanium, plomb et indium, la teneur globale en métaux M des catalyseurs renfermant de l'étain et au moins un autre métal M étant d'environ 0,01 à 1,20 % et de préférence 0,02 à 1,0 % et plus particulièrement 0,03 à 0,80 %.

Les exemples suivants illustrent l'invention sans en limiter la portée.

## EXEMPLE 1

On se propose de transformer du propane en présence d'un catalyseur à base d'un mélange d'une zéolithe MFI de rapport Si/Al = 45 et d'une alumine contenant du platine et un métal choisi dans le groupe constitué par l'étain, le germanium, l'indium et le plomb.

### Préparation de la zéolithe MFI

La zéolithe MFI est synthétisée en présence de structurant organique en utilisant une des recettes connues dans l'art antérieur (US-A-3702886). Cette zéolithe est transformée en une forme H par les traitements suivants :
- calcination sous un mélange air-azote (10 % d'oxygène dans le mélange à 550°C pendant 4 heures,
- trois échanges dans $NH_4NO_3$ 5N à 100°C,
- calcination sous air à 530°C pendant 5 heures sous un débit de 5l/h/g.

Le rapport Si/Al de la zéolithe HMFI est égal à 45, son volume poreux mesuré par adsorption d'azote à 77K supérieur à 0,160 cm³/g.

Préparation de l'alumine renfermant les métaux choisis

Préparation d'une alumine A renfermant 0,30 % de platine en poids.

L'alumine A est préparée en ajoutant à 100 grammes d'alumine (de surface spécifique 240 m2/g et de volume poreux 0,58 cm³/g) 100 cm³ d'une solution d'acétyl acétonate de platine dans le toluène. La concentration en platine de cette solution est égale à 3 grammes par litre.

On laisse 6 heures en contact, on essore, on sèche 1 heure à 100-120°C puis on calcine 2 heures à 530°C. Puis on réduit sous courant d'hydrogène sec pendant 2 heures à 450°C.

De la même façon on prépare une alumine A renfermant en poids 0,60% de platine.

Préparation d'une alumine H renfermant 0,30 % de platine et 0,30 % d'étain.

Le catalyseur H est préparé en ajoutant à 100 grammes d'alumine 100 cm³ d'une solution d'acétylacétonate de platine dans le toluène. La concentration de platine de cette solution est égale à 3 g/litre.

On laisse en contact 6 heures, on essore, on sèche une heure à 120 % puis on calcine 2 heures à 530°C sous air sec.

On procède alors à la fixation de l'étain de la façon suivante : une solution aqueuse d'acétate d'étain est mise en contact avec le support d'alumine à raison de 100 cm³ de solution pour 100 grammes de l'alumine précédente, pendant 6 heures. Le solide obtenu est alors essoré et séché 1 heure à 100-120°C puis calciné à 530°C.

EXEMPLE 2

On a testé la MFI préparée ci-dessus ainsi que ses mélanges avec les alumines préparées ci-dessus utilisées dans un test de transformation du propane dans les conditions suivantes :
- température :    450°C
- pression :    atmosphérique
- pph :    0,5 h$^{-1}$
- charge :    $C_3H_8$

TABLEAU I

| Catalyseur | Conversion (% moles) | Sélectivité (% moles) | | | | |
|---|---|---|---|---|---|---|
| | | $CH_4$ | $C_2H_6$ | $C_2H_4$ | $C_3H_6$ | Aromatiques |
| MFI | 14 | 45 | 15 | 10 | 30 | 0 |
| Mélange 25 % MFI + 75 % A (alumine et 0,30 % Pt) | 15 | 35 | 30 | 5 | 20 | 10 |
| Mélange 25 % MFI + 75 % A (alumine et 0,60 % Pt) | 18 | 33 | 30 | 5 | 20 | 12 |
| Mélange 25 % MFI + 75 % H (alumine et Pt+Sn) | 20 | 7 | 18 | 5 | 40 | 30 |

**Revendications**

1. Procédé catalytique d'aromatisation d'hydrocarbures renfermant 2 à 4 atomes de carbone par molécule en présence d'un catalyseur renfermant (a) une zéolithe MFI de rapport Si/Al supérieur à 7 et (b) un support renfermant au moins un métal noble de la famille du platine, le platine étant toujours présent, et au moins un métal additionnel choisi dans le groupe constitué par l'étain, le germanium, le plomb et l'indium.

2. Procédé selon la revendication 1 dans lequel le catalyseur renferme en poids :
   a) 1 à 99 % de zéolithe MFI,
   b) 99 à 1 % d'un support renfermant 0,01 à 2% d'au moins un métal de la famille du platine, le platine étant toujours présent, et au moins un métal additionnel, dans la proportion de 0,005 à 0,60 % lorsque celui-ci est l'étain ou de 0,005 à 0,70 % lorsque celui-ci est le germanium, le plomb ou l'indium.

EP 0 457 640 B1

3. Procédé selon l'une des revendications 1 et 2 dans lequel le support est l'alumine.

## Claims

1. Catalytic process for the aromatization of hydrocarbons containing 2 to 4 carbon atoms per molecule in the presence of a catalyst containing (a) a zeolite MFI having a Si/Al ratio higher than 7 and (b) a support containing at least one noble metal from the platinum group and at least one additional metal chosen from the group constituted by tin, germanium, lead and indium.

2. Process according to claim 1, wherein the catalyst contains by weight:
   a) 1 to 99% zeolite MFI,
   b) 99 to 1% of a support containing 0.01 to 2% of a metal from the platinum group, platinum being always present, and an additional metal, in the proportion of 0.005 to 0.60% when the latter is tin or 0.005 to 0.70% additional metal when the latter is germanium, lead or indium.

3. Process according to any one of the claims 1 and 2, wherein the support is alumina.

## Patentansprüche

1. Katalytisches Verfahren zur Aromatisierung von Kohlenwasserstoffen mit 2 bis 4 Kohlenstoffatomen pro Molekül in Gegenwart eines Katalysators, der enthält
   a) einen MFI-Zeolithen mit einem Si/Al-Verhältnis >7 und
   b) einen Träger, der mindestens ein Edelmetall aus der Platin-Gruppe, wobei das Platin stets vorhanden ist, und mindestens ein zusätzliches Metall, ausgewählt aus der Gruppe, die besteht aus Zinn, Germanium, Blei und Indium, enthält.

2. Verfahren nach Anspruch 1, wobei der Katalysator, bezogen auf das Gewicht, enthält:
   a) 1 bis 99 % MFI-Zeolith und
   b) 99 bis 1 % eines Trägers, der 0,01 bis 2 % mindestens eines Metalls aus der Platingruppe, wobei das Platin stets vorhanden ist, und mindestens ein zusätzliches Metall in einem Mengenanteil von 0,005 bis 0,60 %, wenn es sich dabei um Zinn handelt, oder von 0,005 bis 0,70 %, wenn es sich dabei um Germanium, Blei oder Indium handelt, enthält.

3. Verfahren nach einem der Ansprüche 1 und 2, wobei es sich bei dem Träger um Aluminiumoxid handelt.

6